# EUROPÄISCHE PATENTSCHRIFT

(11) **EP 1 031 566 B1**
(45) Veröffentlichungstag und Bekanntmachung des Hinweises auf die Patenterteilung: **25.06.2003**
(21) Anmeldenummer: 00102243.3
(22) Anmeldetag: 14.02.2000
(51) Int. Cl.: C07D 277/32

(54) **Verfahren zur Herstellung von 2-Chlor-5-chlormethylthiazol**
Process for the preparation of 2-chloro-5-chloromethyl-thiazole
Procédé pour la préparation du 2-chloro-5-chlorométhyle-thiazole

(30) Priorität: 26.02.1999 DE 19908447
(43) Veröffentlichungstag der Anmeldung: 30.08.2000
(73) Patentinhaber: Bayer CropScience AG, 40789 Monheim (DE)
(72) Erfinder: Decker, Matthias, Dr., 50674 Köln (DE)

(56) Entgegenhaltungen:
- EP-A- 0 446 913
- EP-A- 0 794 180

## Beschreibung

Die Erfindung betrifft ein neues Verfahren zur Herstellung von 2-Chlor-5-chlormethylthiazol.

Es ist bekannt, daß man 2-Chlor-5-chlormethylthiazol erhält, wenn man Allylisothiocyanat (Allylsenföl) der Formel (A) mit einem Chlorierungsmittel gemäß dem folgenden Reaktionsschema umsetzt: 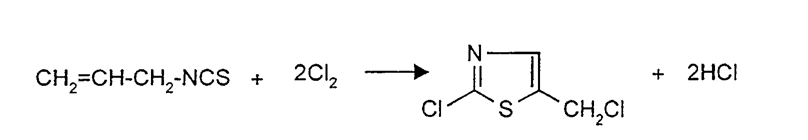 (vgl. hierzu EP-A 0260560).

Ferner ist bekannt, daß man 2-Chlor-5-chlormethylthiazol erhalten kann, wenn man Allylisothiocyanate der Formel (B) mit einem Chlorierungsmittel gemäß dem folgenden Reaktionsschema umsetzt: 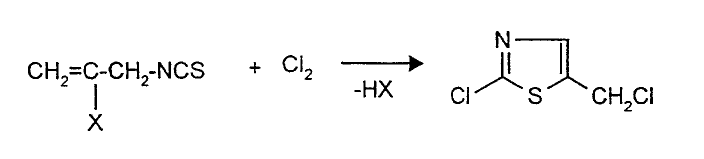 X = Abgangsgruppe
(vgl. hierzu EP-A 0 446 913).

Diese Verfahren haben jedoch den Nachteil, daß ein beträchtlicher Überschuß an Chlorierungsmittel verwendet wird, in großer Verdünnung gearbeitet werden muß und eine hohe Reaktionstemperatur erforderlich ist.

Darüber hinaus muß die sich im Reaktionsverlauf bildende stabile Zwischenstufe in einem zusätzlichen Reaktionsschritt exotherm in das gewünschte Endprodukt überführt werden. Das erfordert, insbesondere bei der Durchführung im technischen Maßstab einen zusätzlichen Überwachungsaufwand.

Desweiteren wird in EP 0 763 531, EP 0 794 180 und WO 98/45279 die Chlorierung von 3-Chlorallylisothiocyanat beschrieben.

Nachteil dieser Verfahren ist die Bildung von Nebenprodukten, die eine aufwendige Reinigung durch Destillation oder Umkristallisation erfordern.

Grundsätzlich andere Verfahren zur Herstellung von 2-Chlor-5-chlormethylthiazol sind in WO 97/10226, EP 0 775 700, EP 0 780 384, WO 97/23469, WO 98/32747 und DE 196 53 586 beschrieben.

Nachteile dieser Verfahren sind zum einen, daß sie von schlecht zugänglichen Verbindungen ausgehen, zum anderen haben sie schlechte Ausbeuten und erfordern teilweise komplizierte Reinigungsverfahren oder Reaktionen mit niedrigem Umsatz.

Es wurde nun gefunden, daß man 2-Chlor-5-chlormethyl-thiazol der Formel (I) 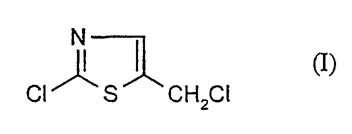 in guten Ausbeuten und hoher Reinheit erhält, wenn man 2-Halogen-alkylisothiocyanat der Formel (II) 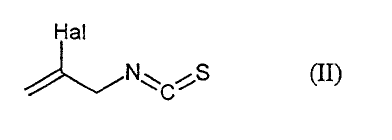 in welcher
- Hal: für Chlor oder Brom steht
mit einem Chlorierungsmittel in Gegenwart eines dipolaren, aprotischen Verdünnungsmittels bei niedrigen Temperaturen umsetzt.

Überraschenderweise kann nach dem erfindungsgemäßen Verfahren das 2-Chlor-5-chlormethyl-thiazol der Formel (I) in sehr guten Ausbeuten und in so hoher Reinheit erhalten werden, daß die erhaltene Rohlösung direkt in Folgereaktionen (Herstellung von Insektiziden, siehe EP-A 0 192 060) eingesetzt werden kann.

Überraschenderweise läßt sich das Verfahren erfolgreich bei niedrigen Temperaturen durchführen, obwohl im Stand der Technik (EP-A 0 446 913) für ein ähnliches Verfahren bei niedrigen Temperaturen der Abbruch der Reaktion auf einer Zwischenstufe beschrieben wird.

Durch den Einsatz des erfindungsgemäßen Verfahrens wird somit der Überwachungsaufwand verringert, da in einem Schritt, bei niedrigen Temperaturen, umgesetzt werden kann. Außerdem entfällt eine aufwendige Reinigung des Produkts, da es in sehr hoher Reinheit anfällt und direkt in Folgereaktionen eingesetzt werden kann.

Verwendet man beispielsweise 2-Chlorallylisothiocyanat als Ausgangsstoff und elementares Chlor als Chlorierungsmittel, so kann der Reaktionsablauf des erfindungsgemäßen Verfahrens durch das folgende Formelschema skizziert werden:

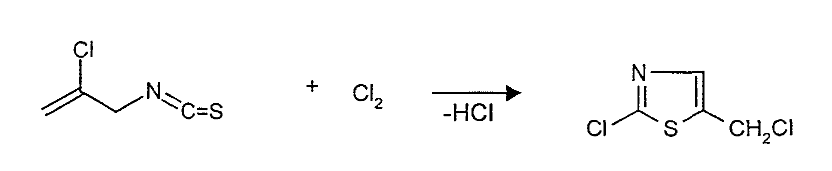

Die beim erfindungsgemäßen Verfahren als Ausgangsstoffe zu verwendenden 2-Halogenallylisothiocyanate sind durch die Formel (II) allgemein definiert. In der Formel (II) steht Hal vorzugsweise für Chlor oder Brom.

Die 2-Halogenallylisothiocyanate sind allgemein bekannt, oder lassen sich nach bekannten Methoden herstellen (siehe EP-A 0 446 913).

Als Chlorierungsmittel kommen elementares Chlor sowie bei den Reaktionsbedingungen chlorabspaltende Verbindungen infrage, wie z.B. Sulfurylchlorid oder Phosgen. Dabei wird das Edukt mit 0,8 bis 2 Äquivalenten, vorzugsweise 1,0 bis 1,5 Äquivalenten, besonders bevorzugt 1,15 bis 1,40 Äquivalenten des Chlorierungsmittels umgesetzt.

Das erfindungsgemäße Verfahren wird in Gegenwart eines dipolaren, aprotischen Lösungsmittels, vorzugsweise Acetonitril, Dimethylformamid, Dimethylsulfoxid, insbesondere Acetonitril, durchgeführt.

Dabei wird mit einem Verhältnis von 1 bis 20 Teilen, vorzugsweise 2 bis 4 Teile, Lösungsmittel auf ein Teil Edukt gearbeitet.

Die Reaktionstemperaturen können bei der Durchführung des erfindungsgemäßen Verfahrens in einem größeren Bereich variiert werden. Im allgemeinen arbeitet man bei Temperaturen zwischen -70°C und 25°C, vorzugsweise bei Temperaturen zwischen -10°C und 20°C, insbesondere bei 10 bis 15°C.

Die Reaktion ist auch bei vermindertem oder erhöhtem Druck möglich, wird aber vorzugsweise bei Normaldruck durchgeführt.

Nach einer Nachrührzeit bei 20°C und nach Entgasen der Reaktionslösung erhält man bereits eine Lösung von 2-Chlor-5-chlormethylthiazol, die nur noch wenig Nebenkomponenten enthält (nach Abzug des Lösungsmittelsanteils liegt das Produkt in 92 bis 94 %iger Reinheit vor).

Diese Lösung kann daher direkt in die folgenden Reaktionen der jeweiligen Wirkstoffsynthese umgesetzt werden.

Aufgrund der hohen Reinheit des 2-Chlor-5-chlormethylthiazol im Reaktionsgemisch kann es auch einfach isoliert werden.

Nach Abkühlen des Reaktionsgemisches auf -10°C bis -50°C, vorzugsweise auf -15°C bis -20°C, kristallisiert 2-Chlor-5-chlormethylthiazol Hydrochlorid aus und kann abfiltriert werden. Aus dem mit kaltem Lösungsmittel gewaschenen Kristallisat wird durch Zugabe von Wasser bei 20°C bis 50°C, vorzugsweise bei 30°C bis 40°C, 2-Chlor-5-chlormethylthiazol freigesetzt und kann als untere flüssige Phase abgetrennt werden.

Nach Waschen mit weiterem Wasser und Trocknen der organischen Phase erhält man 2-Chlor-5-chlormethylthiazol in guter Ausbeute und hoher Reinheit (94 %iges Produkt in 71 % Ausbeute).

Eine weitere Möglichkeit der Aufarbeitung besteht darin, nach Entgasen des Reaktionsansatzes unter schonenden Bedingungen (30 bis 40°C, 20 bis 200 mbar) das Lösungsmittel abzudestillieren und den Destillationsrückstand zum Entfernen der Lösungsmittel und Säurereste mit Wasser zu waschen.

Wird beim erfindungsgemäßen Verfahren anstelle eines Chlorierungsmittels ein entsprechendes Bromierungsmittel eingesetzt, erhält man das 2-Brom-5-brommethylthiazol der Formel (III)

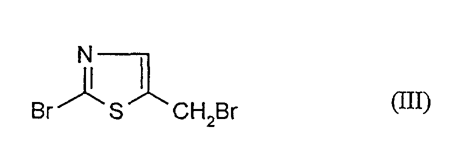

Das 2-Brom-5-brommethylthiazol der Formel (III) ist bekannt (vgl. EP-A 0 376 279).

Das nach dem erfindungsgemäßen Verfahren herzustellende 2-Chlor-5-chlormethylthiazol der Formel (I) kann als Zwischenprodukt zur Herstellung von biologisch aktiven Verbindungen, beispielsweise von Insektiziden verwendet werden (vgl. z.B. EP-A 0 192 060).

### Herstellungsbeispiele

### Beispiel 1

580 g (4,0 mol) 2-Chlorallylisothiocyanat werden in 860 g Acetonitril gelöst. Bei 10 bis 15°C werden 390 g (5,5 mol) Chlor eingeleitet und bei 20 bis 25°C wird 2 Stunden nachgerührt. Der Ansatz wird auf -10°C abgekühlt und eine Stunde bei dieser Temperatur nachgerührt. Das dabei entstandene Kristallisat wird mit kalten Acetonitril gewaschen.

Das Kristallisat wird mit 2 kg 40°C warmem Wasser versetzt, dabei bilden sich zwei flüssige Phasen. Die Phasen werden getrennt, die organische Phase wird bei 30 bis 35°C mit 400 g Wasser gewaschen und bei 30°C im Vakuum getrocknet.

Man erhält 507 g (2,84 mol) 2-Chlor-5-chlormethylthiazol als Schmelze bei einem Gehalt von 94 % (GC, iStd.); das entspricht einer Ausbeute von 71 % der Theorie.

### Beispiel 2

560 g (4,0 mol) 2-Chlorallylisothiocyanat werden wie in Beispiel 1 eingesetzt. Nach der Reaktion wird zwei Stunden bei 20 bis 25°C nachgerührt. Anschließend wird der Ansatz bei 30 bis 35°C im Vakuum entgast.

Man erhält 1838 g einer 34 %igen (GC, iStd.) Lösung von 2-Chlor-5-chlormethylthiazol in Acetonitril; das entspricht einer Ausbeute von 93 % der Theorie.

Nach Abzug des Lösungsmittelgehalts hat das Produkt eine Reinheit von 96 %.

### Beispiel 3

560 g (4,0 mol) 2-Chlorallylisothiocyanat werden wie in Beispiel 1 eingesetzt. Nach der zweistündigen Nachrührzeit bei 20 bis 25°C werden bei 30 bis 35°C der Großteil des Acetonitrils, ca. 750 g, im Vakuum abdestilliert.

Der Rückstand wird bei 30 bis 35°C mit 4 Wasser versetzt, die Phasen werden getrennt und die organische Phase wird mit 400 g Wasser nachgewaschen und bei 30°C im Vakuum getrocknet.

Man erhält 635 g 2-Chlor-5-chlormethylthiazol in einer Reinheit von 92 % (GC, iStd.), das entspricht einer Ausbeute von 87 % der Theorie.

## Patentansprüche

1. Verfahren zur Herstellung von 2-Chlor-5-chlormethylthiazol der Formel (I) 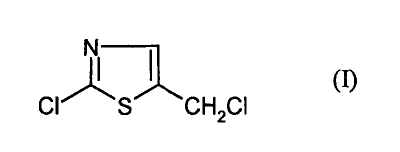 durch Umsetzung von 2-Halogenallylisothio**c**yanaten der Formel (II) 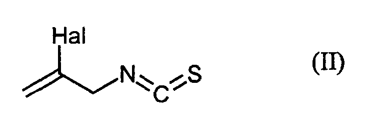 in welcher
Hal für Brom oder Chlor steht,
mit einem Chlorierungsmittel,
**dadurch gekennzeichnet, dass** man die Reaktion in Gegenwart eines dipolaren, aprotischen Lösungsmittels durchführt.

2. Verfahren gemäß Anspruch 1, **dadurch gekennzeichnet, dass** das dipolar, aprotische Lösungsmittel aus der Reihe Acetonitril, Dimethylformamid und Dimethylsulfoxid ausgewählt ist.

3. Verfahren gemäß Anspruch 1 oder 2, **dadurch gekennzeichnet, dass** man die Reaktion in einem Schritt bei Temperaturen zwischen -70°C und +2**5**°C durchführt.

## Claims

1. Process for preparing 2-chloro-5-chloromethylthiazole of the formula (I) 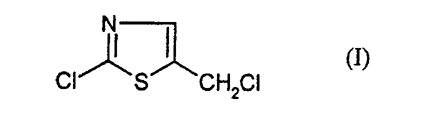 by reacting 2-haloallyl isothiocyanates of the formula (II) 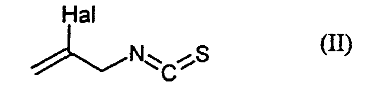 in which
Hal represents bromine or chlorine
with a chlorinating agent,
**characterized in that** the reaction is carried out in the presence of a dipolar aprotic solvent.

2. Process according to Claim 1, **characterized in that** the dipolar aprotic solvent is selected from the group consisting of acetonitrile, dimethylformamide and dimethyl sulphoxide.

3. Process according to Claim 1 or 2, **characterized in that** the reaction is carried out in one step at temperatures between -70°C and +25°C.

## Revendications

1. Procédé de production du 2-chloro-5-chlorométhyl-thiazole de formule (I) 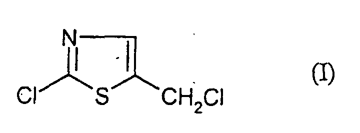 par réaction d'isothiocyanates de 2-halogénallyle de formule (II) 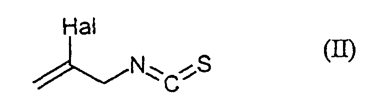 dans laquelle
Hal représente le brome ou le chlore,
avec un agent de chloration,
**caractérisé en ce qu'**on conduit la réaction en présence d'un solvant aprotique dipolaire.

2. Procédé suivant la revendication 1, **caractérisé en ce que** le solvant aprotique dipolaire est choisi dans la série de l'acétonitrile, du diméthylformamide et du diméthylsulfoxyde.

3. Procédé suivant la revendication 1 ou 2, **caractérisé en ce que** la réaction est conduite en une seule étape à des températures comprises entre -70°C et +25°C.
